# EUROPEAN PATENT APPLICATION

(11) **EP 4 647 768 A1**
(43) Date of publication of application: **12.11.2025**
(21) Application number: 23914998.2
(22) Date of filing: 27.12.2023
(51) Int. Cl.: G01N 33/53, G01N 33/543, G01N 33/80, B01L 3/00, G01N 21/84

(54) **ANALYSIS DEVICE HAVING FUNCTION OF CORRECTING ANALYTE CONCENTRATION ACCORDING TO HEMATOCRIT LEVEL OF WHOLE BLOOD SAMPLE**

(30) Priority: 05.01.2023 KR 20230001739
(71) Applicant: SD Biosensor, Inc., Gyeonggi-do 16690 (KR)
(72) Inventor: HONG, Woo Gyeong, Suwon-si, Gyeonggi-do 16690 (KR); PARK, Jiyeong, Suwon-si, Gyeonggi-do 16690 (KR); CHOI, Hyoung-Gil, Suwon-si, Gyeonggi-do 16690 (KR); PARK, Hyo-Lim, Suwon-si, Gyeonggi-do 16690 (KR); KO, Hyungeun, Suwon-si, Gyeonggi-do 16690 (KR)
(74) Representative: J A Kemp LLP
(86) International application number: PCT/KR2023/021638
(87) International publication number: WO 2024/147538

(57) **Abstract**

The present invention relates to an analysis apparatus having the function of correcting the concentration of an analyte according to the hematocrit value of a whole blood sample.

## Description

### [Technical Field]

The present disclosure relates to an analysis apparatus having the function of correcting the concentration of an analyte according to the hematocrit value of a whole blood sample.

### [Background Art]

Whole blood refers to blood that has not been separated into plasma or serum.

Since the process of separating plasma or serum from whole blood requires much time and work, technology development for measuring the concentration of analytes contained in whole blood using whole blood has been actively conducted recently.

Meanwhile, hematocrit (HCT) is the volume of red blood cells in whole blood expressed as a percentage, and is also called red blood cell volume. It is one of the biomarkers used to diagnose anemia/dehydration.

As a result of multiple experiments by the present inventors, a phenomenon has been observed: the higher the HCT value, i.e., the greater the red blood cell volume contained in whole blood, the lower the measured analyte concentration, even in a sample having the same analyte concentration.

It has been confirmed that red blood cells in whole blood act as interference substances in the measurement of analytes, and various technologies have been developed to solve the problem of decreased analyte concentration accuracy due to interference substances.

Korean Patent Laid-open Publication No. 10-2013-0041795 discloses an apparatus having the function of correcting the concentration of a measured analyte according to a HCT value as an apparatus capable of measuring both a HCT value and the concentration of an analyte. The 3^{rd} pad of a strip is provided with a substance for removing unnecessary components for the measurement of the analyte, so that the problem of the accuracy of the analyte concentration measurement being lowered due to unnecessary components, can be prevented.

However, in Korean Patent Laid-open Publication No. 10-2013-0041795, a whole blood hemolysis process is necessary, and the cumbersome task of diluting by 50 to 400 times is required.

Thus, the present inventors have devised and developed the present invention, in which a hemolysis process and a high-magnification dilution process are not required, and the concentration of an analyte can be corrected according to a HCT value.
(Patent Document 1) Korean Patent Laid-open Publication No. 10-2013-0041795 (April 25, 2013)
(Patent Document 2) Japanese Patent Registration No. 6103776 (March 10, 2017)

### [Disclosure]

### [Technical Problem]

Accordingly, the present disclosure provides an analysis apparatus in which analyte concentration correction is performed according to a hematocrit (HCT) value in a sample so that the influence of interference on analyte concentration measurement due to interference substances can be eliminated as much as possible.

The present disclosure also provides a strip with high accuracy of analyte measurement (without the need for red blood cell hemolysis) by treating a first material that captures red blood cells on all of a sample pad, a conjugate pad, and a reserve pad to prevent a decrease in sensitivity at low analyte concentrations due to interference from red blood cells in a sample when the use of whole blood as a sample is required, so that no red blood cell flow is observed on a membrane.

### [Technical Solution]

According to an embodiment of the present disclosure, there is provided a strip including: a sample pad on which a sample is placed and a first material for capturing red blood cells is treated; a conjugate pad, which is provided downstream of the sample pad and on which the first material is treated and a second material that is specifically coupled to an analyte and a third material that migrates together as the sample migrates, are provided; a reserve pad, which is provided downstream of the conjugate pad and on which the first material is treated; a membrane, which is provided downstream of the reserve pad and includes an inspection line in which a fourth material that is specifically coupled to the analyte is fixed, and a control line in which a fifth material that is specifically coupled to the third material is fixed; and an absorbent pad provided downstream of the membrane.

The sample placed on the sample pad may include whole blood and may be a whole blood sample that is diluted with a buffer in an amount two times or more but 50 times or less the amount of the whole blood and is not hemolyzed.

The sample placed on the sample pad may be a whole blood sample that is diluted with a buffer in an amount two times or more but 10 times or less the amount of the whole blood and is not hemolyzed.

The treatment concentration of the first material may be highest on the sample pad and may be lowest on the conjugate pad.

The first material may include lectin and anti-human red blood cells (RBCs).

The strip may further include an external housing including holes through which partial regions of the sample pad, the reserve pad, and the membrane are exposed to the outside.

According to another embodiment of the present disclosure, there is provided an analysis apparatus for analyzing a sample placed on the above-described strip, the analysis apparatus including: a case having an insertion port into which the strip is inserted, and a display for outputting an analysis result; an optical apparatus which is formed inside the case and includes a light emitter for radiating light toward the strip and a detector for collecting light reflected from the strip; an analysis apparatus calculating a hematocrit value of the sample placed on the strip and an analyte concentration using reflected light collected by the detector; and a correction apparatus correcting the analyte concentration according to the hematocrit value and calculating a corrected analyte concentration.

The optical apparatus may include: a first light emitter radiating light in a visible ray range to a region including a reserve pad of the strip; a first detector collecting light that is radiated by the first light emitter and reflected from the strip; a second light emitter radiating light in an ultraviolet ray range to a region including an inspection line of a membrane pad of the strip; and a second detector collecting light that is radiated by the second light emitter and reflected from the strip.

The analysis apparatus may calculate the hematocrit value using optical signals collected by the first detector and calculate the analyte concentration using optical signals collected by the second detector.

The corrected analyte concentration calculated by the correction apparatus and the hematocrit value calculated by the analysis apparatus may be output through the display.

The analyte may be one or more selected from the group consisting of procalcitonin, troponin, and creatine kinase-MB.

### [Advantageous Effects]

According to the present invention, analyte concentration correction is performed according to a hematocrit (HCT) value in a sample so that the influence of interference on analyte concentration measurement due to interference substances can be eliminated as much as possible.

In addition, even when whole blood diluted at a low concentration is used as a sample, a first material that captures red blood cells is treated on all of a sample pad, a conjugate pad, and a reserve pad so that no red blood cell flow is observed on a membrane, and thus the accuracy of analyte measurement is high.

### [Brief Description of the Drawings]

FIGS. 1 and 2 are schematic views for explaining a strip and an external housing according to an embodiment of the present disclosure.
FIG. 3 is a schematic view for explaining the sequence of an analyzing process using the strip and an analysis apparatus.
FIG. 4 is experimental data for measuring a coefficient of variation (CV) for hematocrit (HCT) when PCT-negative specimens with HCT values of 20, 40, and 60 were measured five times repeatedly.
FIG. 5 is experimental data for comparing a control group in which analyte concentration was corrected, with an experimental group in which analyte concentration was corrected according to a HCT value.
FIG. 6 is experimental data for measuring HCT value variability that occurs according to a dilution ratio of whole blood and a buffer.
FIGS. 7 and 8 are views of the results of a verification experiment to verify the superiority of a strip according to an embodiment of the present disclosure.
FIG. 9 is a schematic view for explaining the configuration of an analysis apparatus according to an embodiment of the present disclosure.

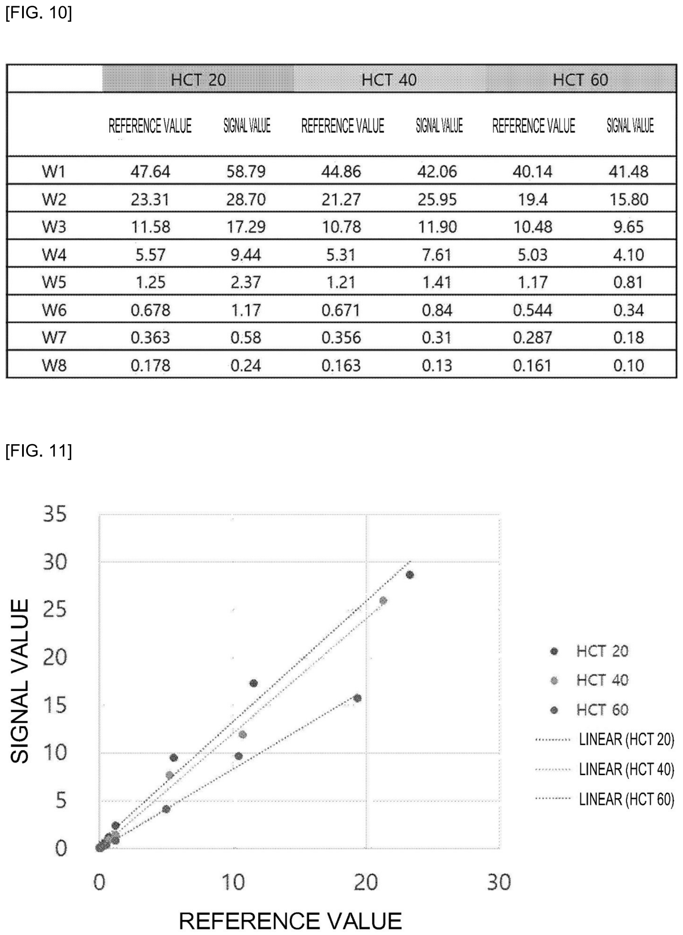

FIGS. 10 and 11 are views for explaining the process of generating a correction formula for calculating a corrected analyte concentration according to a HCT value.

### [Detailed Description of Preferred Embodiments]

In some cases, in order to avoid ambiguity in the concept of the present disclosure, known structures or devices may be omitted or illustrated in block diagram form based on the core functions of each structure and device.

Throughout the specification, when a part is referred to as "comprising" or "including" a certain component, this does not mean excluding other components but further including other components unless specifically stated otherwise. In addition, terms such as "part", "unit", and "module" described in the specification mean a unit that processes at least one function or operation, which may be implemented by hardware or software or a combination of hardware and software. In addition, "a (or an)", "one", "the", and similar related words may be used in the context of describing the present disclosure (especially in the context of the claims below) to include both singular and plural meanings, unless otherwise indicated in the specification or clearly contradicted by the context.

In describing embodiments of the present disclosure, when it is determined that a detailed description of a known function or configuration may unnecessarily obscure the gist of the present disclosure, the detailed description will be omitted. In addition, the terms described below are terms defined in consideration of functions in the embodiments of the present disclosure, and these may vary depending on the intention or custom of a user or operator. Thus, the definitions should be made based on the contents throughout this specification.

Hereinafter, the present disclosure will be described in detail with reference to the accompanying drawings.

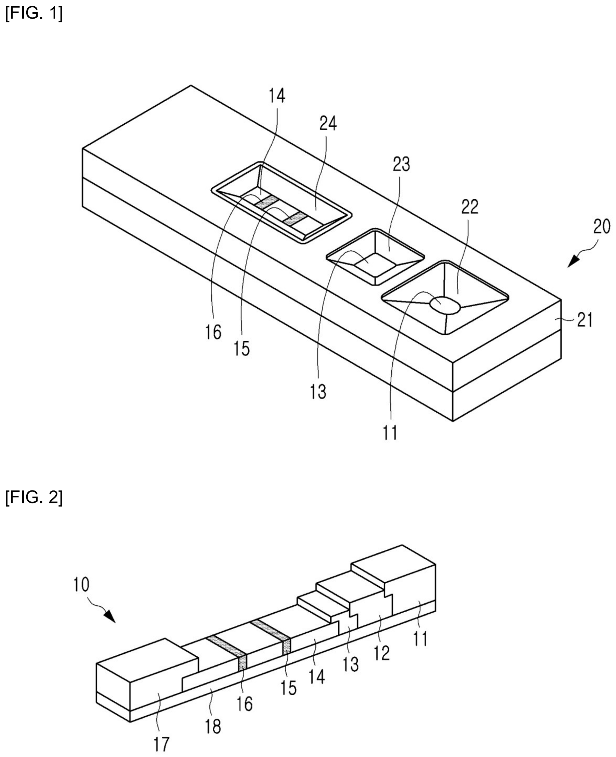

Referring to FIGS. 1 and 2, a strip 10 according to an embodiment of the present disclosure includes a sample pad 11, a conjugate pad 12, a reserve pad 13, a membrane 14, an absorbent pad 17, and a backing card 18.

A sample to be analyzed is placed on the sample pad 11. For example, the sample pad 11 may be manufactured of glass fiber, but the present disclosure is not limited thereto.

A first material that captures red blood cells is treated on the sample pad 11. The first material includes lectin and anti-human red blood cells (RBCs) that specifically capture red blood cells, but the present disclosure is not limited thereto.

The conjugate pad 12 is provided downstream of the sample pad 11 and is configured to partially overlap the sample pad 11. The sample to be analyzed migrates through the sample pad 11 to the conjugate pad 12.

The conjugate pad 12 may be manufactured of polyester, but the present disclosure is not limited thereto.

A second material that is specifically coupled to an analyte is provided on the conjugate pad 12. Specifically, the second material may be an antibody that includes a fluorescent substance (e.g., europium) that reflects or absorbs light having a specific wavelength, for example, in an ultraviolet ray range, and radiates the reflected light of a specific wavelength to the outside, but the present disclosure is not specifically limited thereto. In addition, the analyte includes procalcitonin, troponin, and creatine kinase-MB, and sepsis, cardiovascular disease, etc. may be diagnosed through the analysis of the concentration of the above-described analyte. However, it is not limited to the above examples, and all substances contained in whole blood may be included in the analyte category of the present disclosure, and diseases that can be diagnosed by the concentration of the analyte may also be included in the diagnostic disease category of the present disclosure.

In addition, a third material, which is specifically coupled to a fifth material fixed to a control line 16 of the membrane 14, is provided on the conjugate pad 12. Specifically, the third material may be an antibody that includes a fluorescent substance (e.g., gold nanoparticles) that reflects or absorbs light having a specific wavelength, for example, in an ultraviolet ray range, and radiates the reflected light of a specific wavelength to the outside, but the present disclosure is not specifically limited thereto.

The first material that captures red blood cells is also treated on the conjugate pad 12. Thus, red blood cells that are not captured in the sample pad 11 are captured in the conjugate pad 12. However, in addition to the first material, the second material and the third material are provided on the conjugate pad 12. The second material is a material that is specifically coupled to the analyte in the sample, and the third material is a material that is specifically coupled to the fifth material fixed to the control line 16. That is, since, in addition to the first material, the second material and the third material are treated on the conjugate pad 12 and the concentration of the analyte is determined according to the amount in which the second material is present in an inspection line 15 and the third material acts as a reference line for calculating analyte concentration, substances other than the second material and the third material are preferably minimally treated on the conjugate pad 12. Thus, the concentration of the first material treated on the conjugate pad 12 is preferably lower than the concentration of the first material treated on the sample pad 11.

The reserve pad 13 is provided downstream of the conjugate pad 12 and is configured to partially overlap the conjugate pad 12. The sample to be analyzed migrates through the conjugate pad 12 to the reserve pad 13.

The reserve pad 13 may be manufactured of polyester, but the present disclosure is not specifically limited thereto.

The first material is also treated on the reserve pad 13 so that red blood cells contained in the sample to be analyzed that has reached the reserve pad 13 through the conjugate pad 12, can be captured. The first material is treated on the reserve pad 13 so that red blood cells that reach the membrane 14 are minimized (preferably, redundant red blood cells are completely captured in the reserve pad so that no red blood cells reach the membrane). Thus, the problem of the accuracy of analyte concentration measurement being lowered due to red blood cells is solved.

The concentration of the first material treated on the reserve pad 13 is lower than that on the sample pad 11 and higher than that on the conjugate pad 12. This is because a significant portion of red blood cells in the sample to be analyzed is captured through the sample pad 11 and the conjugate pad 12, and the reserve pad 13 prevents red blood cells from flowing into the membrane 14 and thus, even when the first material is treated at a concentration lower than that on the sample pad 11, sufficient effects can be achieved.

The membrane 14 is provided downstream of the reserve pad 13 and is configured to partially overlap the reserve pad 13 and the absorbent pad 17 between the reserve pad 13 and the absorbent pad 17 to be described below. The sample to be analyzed migrates through the reserve pad 13 to the membrane 14.

The membrane 14 may be manufactured as a cellulose membrane, but the present disclosure is not specifically limited thereto.

The inspection line 15 in which a fourth material to which the analyte is specifically coupled, is fixed, and the control line 16 in which a fifth material specifically coupled to the third material provided on the conjugate pad 12 is fixed, are formed in the membrane 14. Here, the concepts of a material being provided on the conjugate pad 12 and a material being fixed on the membrane 14 are different. The concept of a material being provided means that a solution injected into the sample pad 11 can move in a direction of migration as it migrates toward the absorbent pad 17, but the concept of a material being fixed means that, even if the solution migrates, it does not move together but is fixed in place.

The analyte contained in the sample migrates in the direction of the absorbent pad 17 and is coupled to the second material provided on the conjugate pad 12, and the analyte is specifically coupled to the fourth material fixed in the inspection line 15. In addition, the sample migrates from the sample pad 11 in the direction of the absorbent pad 17, and the third material provided on the conjugate pad 12 is directed to the control line 16 of the membrane 14. A light emitter of an optical apparatus 54 to be described below radiates light in an ultraviolet ray range toward a region in which the inspection line 15 and the control line 16 are included, and a detector receives the light reflected from the region. An analysis apparatus 55 calculates the concentration of the analyte using light received by the detector of the optical apparatus 54. For example, a method of calculating the concentration of the analyte by comparing the received light of the inspection line 15 with the received light of the control line 16 may be applied.

The absorbent pad 17 is provided downstream of the membrane 14 and is configured to partially overlap the membrane 14. The absorbent pad 17 provides the force by which the sample placed on the sample pad 11 migrates to the absorbent pad 17.

The absorbent pad 17 may be manufactured of natural or synthetic fibers, but the present disclosure is not specifically limited thereto.

The backing card 18 is disposed at a lower portion of the strip 10, serves as a support for the strip 10, and extends the entire length of the strip 10.

The backing card 18 may be formed of polyethylene, but the present disclosure is not specifically limited thereto.

The strip 10 according to the embodiment of the present disclosure may be installed inside the external housing 20.

The external housing 20 is configured to surround the outside, excluding a partial region of the strip 10.

Referring to FIG. 1, the external housing 20 includes an upper external housing 21 and a lower external housing, and holes through which a first region including the inspection line 15 of the strip 10, a second region that is at least a part of the reserve pad 13, and a third region that is at least a part of the sample pad 11 are exposed to the outside, are formed in the upper external housing 21.

Specifically, a first hole 22 for exposing the third region of the sample pad 11 to the outside, a second hole 23 for exposing the second region of the reserve pad 13 to the outside, and a third hole 24 for exposing the first region of the membrane 14 to the outside are formed in the upper external housing 21.

Meanwhile, the first hole 22 is a hole through which the sample is placed, the second hole 23 is a portion through which the optical apparatus 54 radiates light toward the reserve pad 13 for HCT value calculation, and the third hole 24 is a portion through which the optical apparatus 54 radiates light toward the membrane 14 for analyte concentration calculation.

The embodiment of the present disclosure for calculating the concentration of the analyte contained in the sample using the strip 10 may further include a sampler 30, a buffer tube 40, and an analysis apparatus 50.

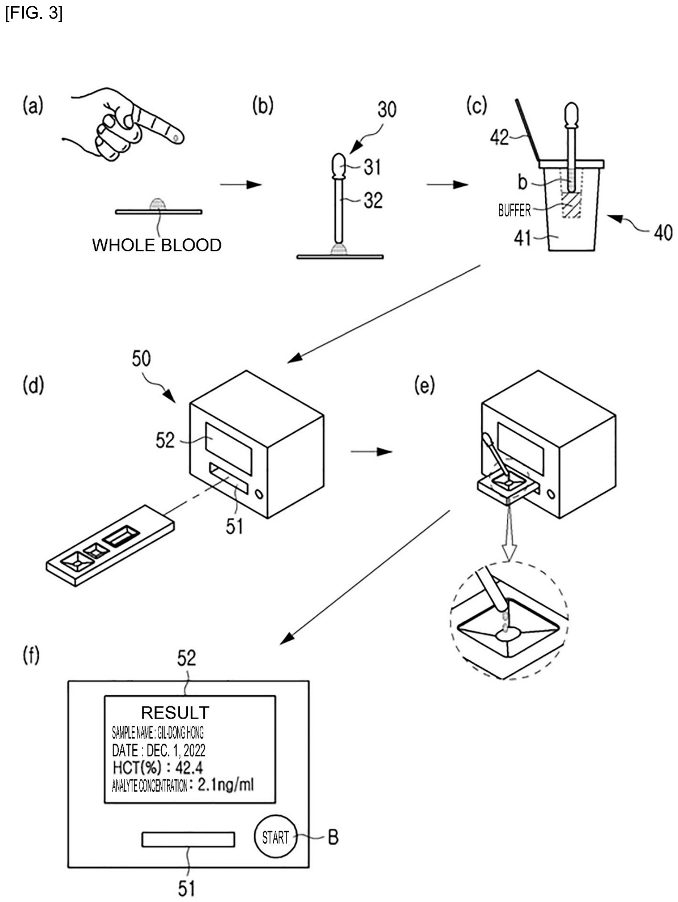

The sampler 30 may be provided in an dropper form, as shown in FIG. 3, and includes a pressurizing head 31 and an extraction rod 32. The pressurizing head 31 and the extraction rod 32 may be manufactured of a soft material, and an empty space is formed in the pressurizing head 31 and the extraction rod 32. While a user pressurizes the pressurizing head 31 and brings the tip of the extraction rod 32 into contact with the sample (whole blood) and then removes the force applied to the pressurizing head 31, the sample is aspirated through the extraction rod 32.

The buffer tube 40 includes a body 41 having a buffer accommodation space therein, and a sealing film 42 that covers an upper opening of the body 41. The user removes the sealing film 42 from the body 41 and discharges the sample aspirated through the sampler 30 into the buffer accommodation space, so that whole blood and the buffer can be mixed with each other.

In particular, the buffer to be mixed with whole blood does not include a material for red blood cell hemolysis, and the amount of the buffer may be, for example, two times or more the sample, in detail, 2.9 times or more the sample, and more specifically, may be two times or more but 100 times or less, specifically, two times or more but 50 times or less, more specifically, two times or more but 25 times or less, two times or more but 20 times or less, two times or more but 15 times or less, two times or more but 10 times or less, or two times or more but 7 times or less. More specifically, 2.9 times or more but 100 times or less, 2.9 times or more but 50 times or less, 2.9 times or more but I 25 times or less, 2.9 times or more but 20 times or less, 2.9 times or more but 15 times or less, 2.9 times or more but 10 times or less, or 2.9 times or more but 7 times or less.

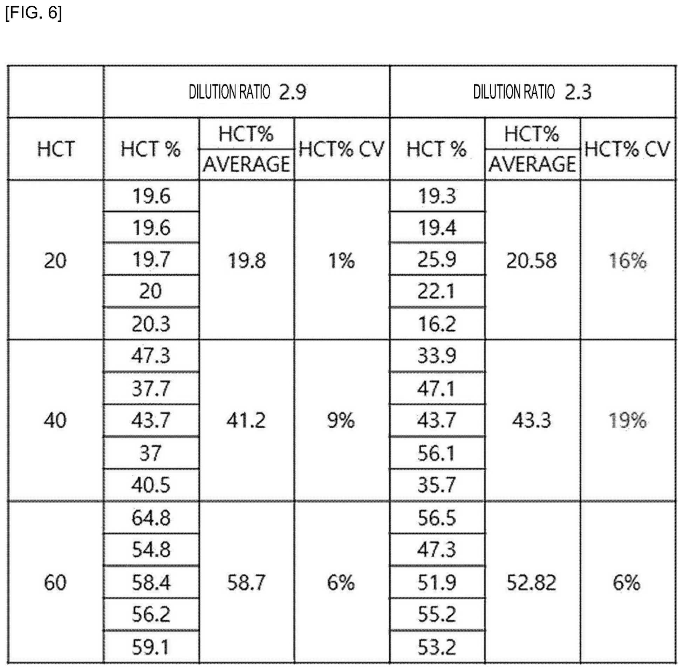

An experiment for measuring the variability of HCT values by setting the dilution ratio of the buffer mixed with whole blood to 2.9 times (experimental group) and 2.3 times (control group), which is lower than 2.9 times, was conducted (see FIG. 6). As a result of the experiment, it was confirmed that the variability was measured to be 10% or less in the experimental group with a dilution ratio of 2.9 times, but the variability was measured to be up to 19% in the control group with a dilution ratio of 2.3 times, which is lower than 2.9 times. As a result of the experiment, it was confirmed that the dilution ratio of whole blood is preferably at least 2.9 times, and it can be clearly understood that the variability of the HCT values will further decrease when the dilution ratio is 2.9 times or more because the red blood cells are more diluted. However, when a whole blood sample is diluted at an excessive ratio, signal intensity may be too weak, which may lower the HCT values and the accuracy of analyte concentration measurement; thus, in the present invention, it is preferable to dilute the whole blood sample with a dilution ratio of 100 times or less.

The sample placed on the strip 10 is a mixture of whole blood and the buffer. Since the material for red blood cell hemolysis is not contained in the buffer, the red blood cells remain in the sample placed on the strip 10. In addition, unlike the case where whole blood was diluted 10,000 to 100,000 times in a strip for measuring hemoglobin concentration according to the related art, in the present invention, the whole blood is diluted two times or more but 100 times or less.

Thus, the concentration of red blood cells contained in the sample placed on the strip 10 is high, and thus, interference may occur in analyte concentration measurement.

In order to prevent interference in analyte concentration measurement by the red blood cells, in the strip 10 according to the embodiment of the present invention, the first material that captures the red blood cells, is treated on the sample pad 11, the conjugate pad 12, and the reserve pad 13. Thus, the red blood cells of the sample placed on the sample pad 11 do not reach the membrane 14 but are captured in the sample pad/conjugate pad/reserve pad.

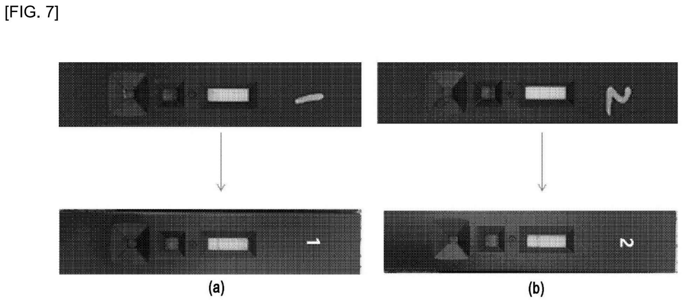

Referring to FIG. 7, result data about an experiment of analyte concentration measurement using (a) the control group, in which the first material is treated only on the conjugate pad 12, and the reserve pad 13 and (b) the experimental group (the present invention), in which the first material is treated on all of the sample pad 11, the conjugate pad 12, and the reserve pad 13, are shown.

In the case of the control group ((a) of FIG. 7), it was confirmed that all red blood cells in the sample were not captured in the conjugate pad 12 and the reserve pad 13, so that the red blood cells flowed into the membrane 14.

On the other hand, in the case of the experimental group ((b) of FIG. 7), it was confirmed that all red blood cells in the sample were captured in the sample pad 11, the conjugate pad 12, and the reserve pad 13, so that the red blood cells did not flow into the membrane 14.

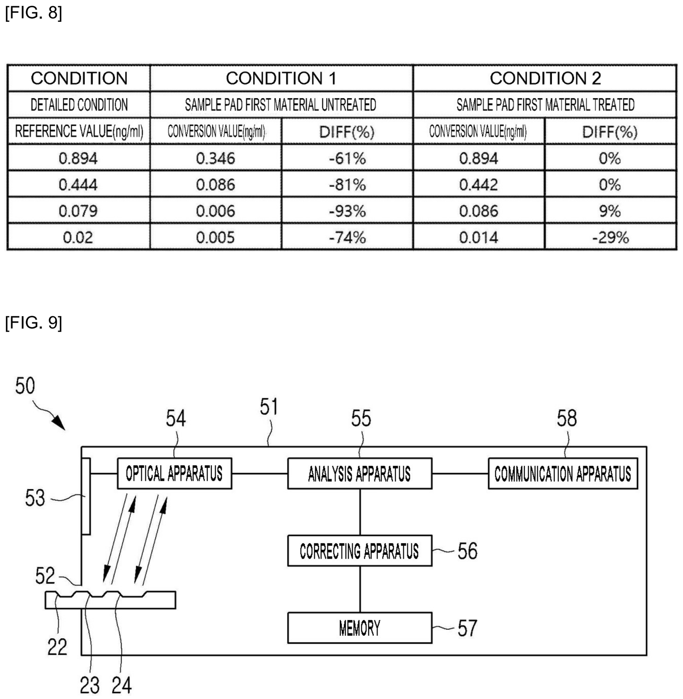

As shown in FIG. 8, in the case of the control group (condition 1), it was confirmed that the red blood cells flowed into the membrane 14, so that the accuracy of analyte concentration measurement was lowered (the absolute value of DIFF(%) was large), but in the case of the experimental group (condition 2), the red blood cells did not flow into the membrane 14, so that the accuracy of analyte concentration measurement was not lowered by the red blood cells.

That is, through the verification experiments conducted in FIGS. 7 and 8, it was confirmed that, when a sample of whole blood diluted at low magnification was applied to the strip, in the experimental group according to the embodiment of the present invention, in which the first material for capturing red blood cells was treated on all of the sample pad/conjugate pad/reserve pad, the accuracy of analyte concentration measurement was not lowered by the red blood cells.

Referring to FIG. 9, the analysis apparatus 50 includes a case 51, an insertion port 52, a display 53, an optical apparatus 54, an analysis apparatus 55, a correction apparatus 56, memory 57, and a communication apparatus 58.

The case 51 is a configuration that constitutes the appearance of the analysis apparatus 50, and the insertion port 52 and the display 53 are formed on a front surface of the case 51.

The strip 10 is inserted into the analysis apparatus 50 through the insertion port 52. In detail, when a third region of the strip 10 is exposed to the outside, the first and second regions of the strip 10 are inserted into the insertion port 52 to be placed inside the case 51. A sample is placed in the third region, and the placed sample migrates to the absorbent pad 17.

The optical apparatus 54 includes a light emitter that radiates light toward the first and second regions, and a detector that receives light reflected from the strip 10.

Specifically, the light emitter radiates light in an ultraviolet ray range (a wavelength range of 337.5 nm to 412.5 nm, more specifically, the wavelength of 375 nm) toward the first region and radiates light in a visible ray range (specifically, a blue range, more specifically, a wavelength range of 424.8 nm to 519.2 nm, and more specifically, the wavelength of 472 nm) toward the second region.

One detector may be provided, but two detectors may also be provided so that each detector may collect light reflected from the first region and light reflected from the second region.

Optical signals collected by the detector are transmitted to the analysis apparatus 55, and the analysis apparatus 55 calculates analyte concentration using the optical signals collected in the first region and HCT value using the optical signals collected in the second region.

A fourth material and a fifth material that emit light of a specific wavelength are placed in the first region according to light radiation in the ultraviolet ray range, and the amount of the fourth material placed in the first region increases in proportion to the amount of the analyte and thus, analyte concentration can be calculated using the optical signals of the specific wavelength.

In addition, red blood cells captured in the first material of the reserve pad 13 are placed in the second region, and the degree of redness varies depending on the number of red blood cells contained in the sample (i.e., according to a HCT value). That is, different optical signals are collected depending on the HCT value in the sample, and these may be used to calculate the HCT value.

The correction apparatus 56 corrects the analyte concentration according to the HCT value calculated in the analysis apparatus 55, thereby calculating the corrected analyte concentration. The HCT value calculated in the analysis apparatus 55 and the corrected analyte concentration calculated in the correction apparatus 56 are output to the outside through the display 53. In addition, the HCT value, the analyte concentration, and the corrected analyte concentration may be transmitted to and stored in the memory 57 and may also be transmitted to an external device through the communication apparatus 58.

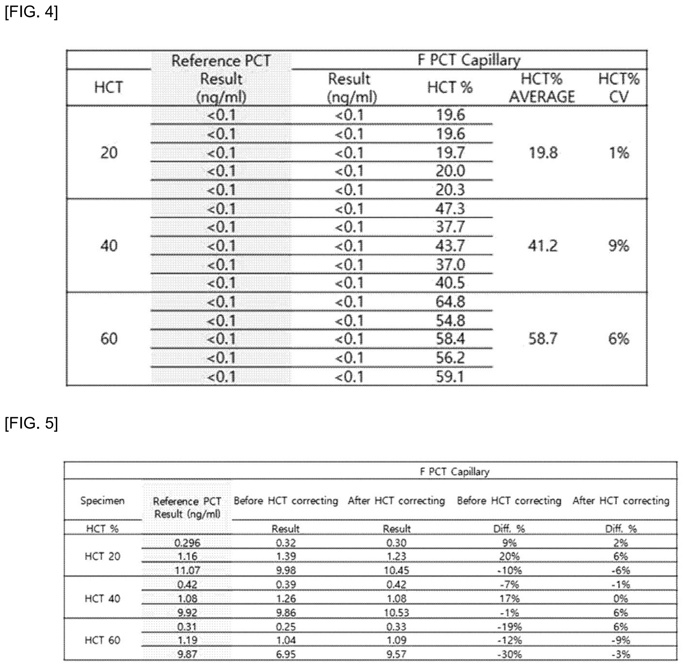

FIG. 5 is experimental data for comparing a control group in which analyte concentration was not corrected, with an experimental group in which analyte concentration was corrected according to a HCT value. It was confirmed that there was a large difference in the accuracy of analyte concentration measurement between the experimental group, in which analyte concentration correction was performed, and the control group, in which analyte concentration correction was not performed, as the analyte concentration at the same HCT value increases or the HCT value increases. In addition, it was confirmed that analyte concentration measurement was possible with an error of 10% or less, in the case of the experimental group in which analyte concentration correction was performed according to the HCT value.

A process of analyte concentration correction according to the HCT value will be briefly described as below.

A correction formula is generated using a graph of signal values for each HCT value (HCT 20, 40, and 60 in FIG. 7) using a plurality of samples having different HCT values/analyte concentration values. Specifically, each formula for correcting analyte concentration according to each HCT values is generated, and the correction apparatus 56 calculates the corrected analyte concentration according to the calculated HCT values using the generated correction formula.

The above-described analysis apparatus 55 and correction apparatus 56 include a processor. The processor may perform a data processing or calculation function, and may store commands or data received from another component in volatile memory, process the commands or data stored in the volatile memory, and store resultant data in nonvolatile memory, as at least part of the data processing or calculation function.

An analysis process using the strip 10 and the analysis apparatus 50 according to the above-described embodiment of the present invention will be described with reference to FIG. 3.

First, whole blood is collected from an object to be analyzed (for example, a human being) ((a) of FIG. 3).

Next, the collected whole blood is aspirated using the sampler 30. Specifically, an end of the extraction rod 32, in a state in which the pressurizing head 31 is pressurized, is in contact with the whole blood, and when pressurizing is released, the whole blood is aspirated into the extraction rod 32 ((b) of FIG. 3).

Next, the sealing film 42 of the buffer tube 40 is removed, and the whole blood aspired into the sampler 30 is placed in the internal space of the buffer tube 40. A buffer is stored in the internal space, and this is used to mix the whole blood with the buffer, so that a dilution process of the whole blood is performed ((c) of FIG. 3). When dilution is completed, a diluted sample is aspirated again through the sampler 30.

Next, the strip 10 is inserted into the insertion port 52 of the analysis apparatus 50 ((d) of FIG. 3).

The strip 10 that has been inserted has a region including the sample pad 11 exposed to the outside, and the diluted sample is placed on the sample pad 11 through the sampler 30 ((e) of FIG. 3).

The sample placed on the sample pad 11 migrates in the direction of the absorbent pad 17, and the corrected analyte concentration and a HCT value (%) of the placed sample are calculated by the optical apparatus 54, the analysis apparatus 55, and the correction apparatus 56 provided inside the analysis apparatus 50 and are output to the outside through the display 53 ((f) of FIG. 3).

While the above description is merely an exemplary description of the technical spirit of the present embodiment, it will be understood by those skilled in the art that various modifications and changes can be made to the present disclosure without departing from the essential characteristics of the present embodiment. Therefore, the protection scope of the present disclosure should be defined by the claims.

### [Explanation of Reference Numerals]

- 10:: strip
- 11:: sample pad
- 12:: conjugate pad
- 13:: reserve pad
- 14:: membrane
- 15:: inspection line
- 16:: control line
- 17:: absorbent pad
- 18:: backing card
- 20:: external housing
- 21:: upper external housing
- 22:: first hole
- 23:: second hole
- 24:: third hole
- 30:: sampler
- 31:: pressurizing head
- 32:: extraction rod
- 40:: buffer tube
- 41:: body
- 42:: sealing film
- 50:: analysis apparatus
- 51:: case
- 52:: insertion port
- 53:: display
- 54:: optical apparatus
- 55:: analysis apparatus
- 56:: correction apparatus
- 57:: memory
- 58:: communication apparatus

## Claims

1. A strip comprising:
a sample pad on which a sample is placed and a first material for capturing red blood cells is treated;
a conjugate pad, which is provided downstream of the sample pad and on which the first material is treated and a second material that is specifically coupled to an analyte and a third material that migrates together as the sample migrates, are provided;
a reserve pad, which is provided downstream of the conjugate pad and on which the first material is treated;
a membrane, which is provided downstream of the reserve pad and comprises an inspection line in which a fourth material that is specifically coupled to the analyte is fixed, and a control line in which a fifth material that is specifically coupled to the third material is fixed; and
an absorbent pad provided downstream of the membrane.

2. The strip of claim 1, wherein the sample placed on the sample pad comprises whole blood and is a whole blood sample that is diluted with a buffer in an amount two times or more but 50 times or less the amount of the whole blood and is not hemolyzed.

3. The strip of claim 2, wherein the sample placed on the sample pad is a whole blood sample that is diluted with a buffer in an amount two times or more but 10 times or less the amount of the whole blood and is not hemolyzed.

4. The strip of claim 2, wherein a treatment concentration of the first material is highest on the sample pad and is lowest on the conjugate pad.

5. The strip of claim 4, wherein the first material comprises lectin and anti-human red blood cells (RBCs).

6. The strip of claim 2, further comprising an external housing comprising holes exposing partial regions of the sample pad, the reserve pad, and the membrane to the outside.

7. An analysis apparatus for analyzing a sample placed on the strip according to any one of claims 1 to 6, the analysis apparatus comprising:
a case having an insertion port into which the strip is inserted, and a display for outputting an analysis result;
an optical apparatus which is formed inside the case and comprises a light emitter for radiating light toward the strip and a detector for collecting light reflected from the strip;
an analysis apparatus calculating a hematocrit value of the sample placed on the strip and an analyte concentration using reflected light collected by the detector; and
a correction apparatus correcting the analyte concentration according to the hematocrit value and calculating a corrected analyte concentration.

8. The analysis apparatus of claim 7, wherein the optical apparatus comprises:
a first light emitter radiating light in a visible ray range to a region including a reserve pad of the strip;
a first detector collecting light that is radiated by the first light emitter and reflected from the strip;
a second light emitter radiating light in an ultraviolet ray range to a region including an inspection line of a membrane pad of the strip; and
a second detector collecting light that is radiated by the second light emitter and reflected from the strip.

9. The analysis apparatus of claim 8, wherein the analysis apparatus calculates the hematocrit value using optical signals collected by the first detector and calculates the analyte concentration using optical signals collected by the second detector.

10. The analysis apparatus of claim 9, wherein the corrected analyte concentration calculated by the correction apparatus and the hematocrit value calculated by the analysis apparatus are output through the display.

11. The analysis apparatus of claim 7, wherein the analyte is one or more selected from the group consisting of procalcitonin, troponin, and creatine kinase-MB.
